(19) Europäisches Patentamt
European Patent Office
Office européen des brevets

(11) Publication number: 0 186 440 A2

# (12) EUROPEAN PATENT APPLICATION

(21) Application number: 85309258.3

(22) Date of filing: 19.12.85

(51) Int. Cl.[4]: C 07 C 69/92
C 07 C 67/39, C 07 C 67/31
C 07 C 93/20

(30) Priority: 26.12.84 PC T/JP84/00617
08.10.85 JP 224028/85

(43) Date of publication of application:
02.07.86 Bulletin 86/27

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Nohara, Akira
15-12, Oharano-kamisatotorimicho
Nishikyo-ku Kyoto 610-11(JP)

(72) Inventor: Maki, Yoshitaka
5-17, Oharano-kamisatotorimicho
Nishikyo-ku Kyoto 610-11(JP)

(74) Representative: Laredo, Jack Joseph et al,
Elkington and Fife High Holborn House 52/54 High
Holborn
London, WC1V 6SH(GB)

## (54) Method for producing benzoic acid derivatives.

(57) Benzoic acid derivatives of the formula:

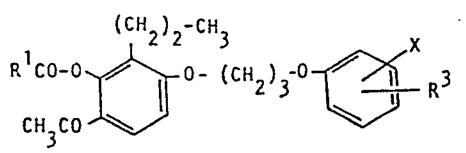

wherein $R^1$ is a lower alkyl group, $R^3$ is -COOH or a group of the formula:

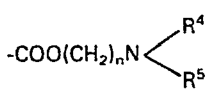

(wherein n is an integer of 2 to 4, and $R^4$ and $R^5$ independently are a hydrogen atom or an alkyl group or they form, together with the adjacent nitrogen atom, a heterocyclic ring) and X is a halogen atom, and of the formula:

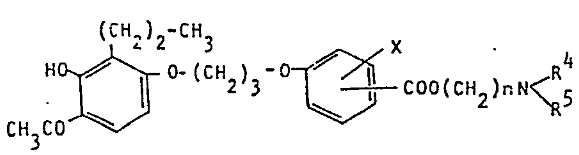

wherein X, n, $R^4$ and $R^5$ are of the same meanings as defined above, which have excellent antiasthmatic and antiinflammatory actions and are useful in the treatment of asthma or inflammatory diseases, can be advantageously produced by subjecting a corresponding aldehyde compound to oxidation or by subjecting corresponding starting compounds to condensation.

EP 0 186 440 A2

Croydon Printing Company Ltd.

# Method for Producing Benzoic Acid Derivatives

This invention relates to a method for producing benzoic acid derivatives exhibiting an antagonistic action on the slow reacting substance of anaphylaxis (SRS-A), which is a group of chemical mediators inducing a contraction of bronchial and other smooth muscles, and being useful as, among others, antiasthmatic agents.

As compounds exhibiting an antagonistic action on SRS-A, there may be mentioned those disclosed in British Patent Application Publication No. 1,384,530, European Patent Application Publication No. 28,063 and European Patent Application Publication No. 80,371.

However, an antagonistic action of those compounds on SRS-A specifically disclosed in working examples of the British Patent Application Publication No. 1,384,530 and European Patent Application Publication No. 28,063 can hardly be satisfactory and an improvement of the action has been desired. As to the compounds disclosed in European Patent Application Publication No. 80,371, an improvement in oral absorbability and an increase in the durability of action have been desired.

The present inventors have diligently conducted research work aiming at obtaining a compound which has a satisfactory antagonistic action on SRS-A, and found that a certain group of benzoic acid derivatives meet the purpose and also found a method for producing the benzoic acid derivatives with an industrial advantage. These findings

were followed by further studies, on which the present invention has been predicated. Thus, the present invention relates to

(1) a method for producing a benzoic acid derivative of the formula:

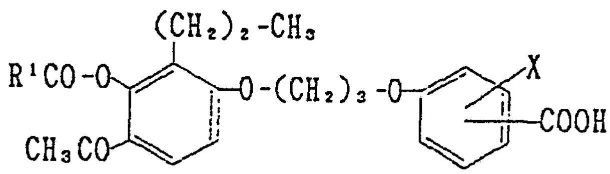

(I)

wherein $R^1$ is a lower alkyl group and X is a halogen atom, which comprises subjecting a compound of the formula:

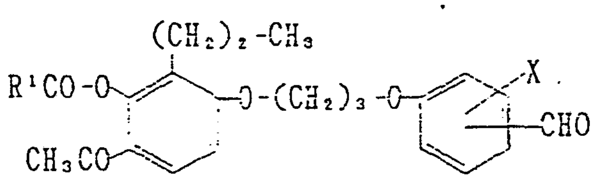

(IV)

wherein $R^1$ and X are of the same meanings as defined above, to oxidation,

(2) a method for producing a benzoic acid derivative of the formula:

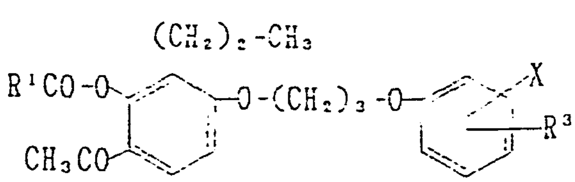

(II)

wherein $R^1$ and X are of the same meanings as defined above, $R^3$ is a carboxyl group which may be protected by a group of the formula:

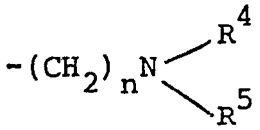 (wherein n is an integer of 2 to 4, $R^4$ and $R^5$ independently are hydrogen or a lower alkyl group, and $R^4$ and $R^5$, taken together with the adjacent nitrogen atom, may form a 5- or 6-membered heterocyclic ring.), which comprises subjecting a compound of the formula:

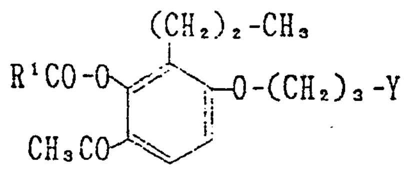

(V)

wherein $R^1$ is of the same meaning as defined above, and Y is a leaving group when condensation is conducted, and a compound of the formula:

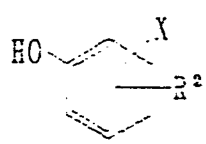

(VI)

wherein $R^2$ is a protected carboxylic group and X is of the same meaning as defined above, to condensation, and then subjecting the thus obtained compound to a reaction for removing the protective group, upon necessity,

(3) a method for producing a benzoic acid derivative of the formula:

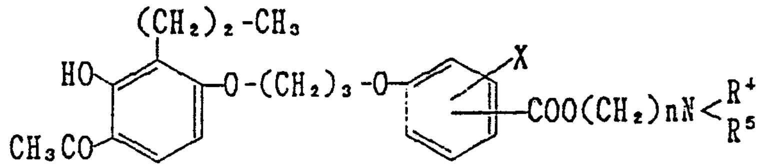

(III)

wherein n, $R^4$, $R^5$ and X are of the same meanings as defined above, which comprises subjecting a compound of the formula:

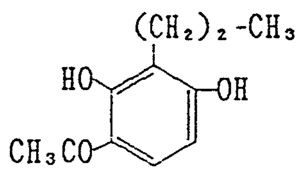

(VII)

and a compound of the formula:

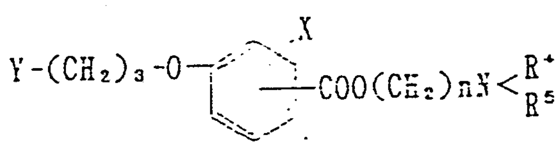

(VIII)

wherein n, $R^4$, $R^5$, X and Y are of the same meanings as defined above, to condensation, and

(4) a method for producing a benzoic acid derivative (III), which comprises subjecting a compound of the formula:

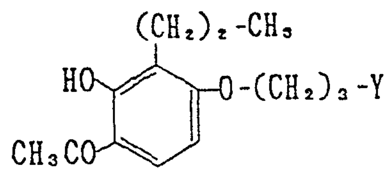 (IX)

wherein Y is of the same meaning as defined above, and a compound of the formula:

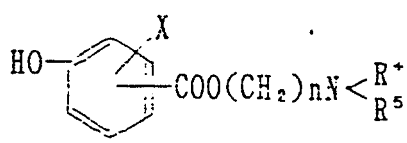 (VI')

wherein n, $R^4$, $R^5$ and X are of the same meanings as defined above, to condensation.

In the above formulae, as the lower alkyl group represented by $R^1$, those having 1 to 3 carbon atoms are preferable, which are exemplified by methyl, ethyl, n-propyl and isopropyl, and especially preferable ones are methyl and ethyl.

In the above formulae, the protective group in the protected carboxyl group represented by $R^2$ and that in the carboxyl group which may be protected represented by $R^3$ is shown by $R^6$, and is exemplified by a group

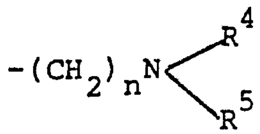 (wherein n, $R^4$ and $R^5$ are of the same meanings as defined above), tetrahydropyranyl, t-butyl, trityl, benzyl, benzyloxymethyl and phenacyl.

In the above formulae, as the lower alkyl group represented by $R^4$ and $R^5$, those having 1 to 3 carbon atoms are preferable, which are exemplified by methyl, ethyl, n-propyl and isopropyl. The five- or six-membered heterocyclic group is such a one as contains one oxygen atom or 1 or 2 nitrogen atoms, which is exemplified by morpholino, piperazino, piperidino or pyrrolidino.

These heterocyclic groups may optionally have one, two or three substituents which are exemplified by $C_{1-3}$ lower alkyl (e.g. methyl, ethyl, n-propyl and isopropyl), $C_{1-3}$ lower alkoxy (e.g. methoxy, ethoxy and propoxy), $C_{1-3}$ acyl (e.g. formyl, acetyl and propionyl) and halogen (e.g. chlorine, bromine, iodine and fluorine).

The halogen atom represented by X is exemplified by bromine, chlorine, fluorine and iodine.

The group represented by the symbol Y, which leaves when the condensation is conducted, is exemplified by halogen (e.g. chlorine, bromine and iodine), p-toluenesulfonyloxy or methanesulfonyloxy.

In the compounds of the present invention,

-CHO, -COOH, $-R^2$, $-R^3$ and 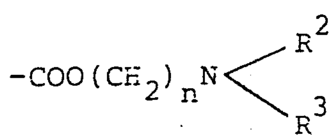 are

respectively located preferably at the para-position, and X is preferably located at the ortho-position.

The reagent to be employed for the reaction in order to obtain the compound (I) by subjecting the compound (IV) to oxidation is exemplified by potassium permanganate, chromium acid, hydrogen peroxide, silver (I) oxide, silver (II) oxide or oxygen. In the reaction, about 1 to 4

equivalents of an oxidizing agent is employed relative to one equivalent of the compound (IV). The solvent to be employed is a conventional organic solvent, especially exemplified by acetic acid, acetone, benzene, acetonitrile or a mixture thereof. Depending on the case, the reaction can be conducted by a two-phase reaction with water. The reaction temperature is normally about 0 to 200°C, preferably about 0 to 50°C and is more preferably around room temperature, while it may be suitably chosen from those ranging from room temperature to the boiling point of the solvent employed. The reaction time depends on the oxidizing agent then employed, and is preferably about 0.5 to 5 hours.

In the respective condensation between the compounds (V) and (VI), the compounds (VII) and (VIII), and the compounds (IX) and (VI'), about 0.5 to 2 equivalents each of the compounds (VI), (VIII) and (VI') are usually employed relative to one equivalent each of the compounds (V), (VII) and (IX). The reaction is conducted preferably in the presence of a base such as potassium carbonate, sodium carbonate, triethylamine, pyridine and 4-dimethylaminopyridine. For conducting the reaction under milder conditions and with a better yield, it is preferable to add potassium iodide or sodium iodide in an amount ranging from catalytic to about equimolar amount. For the reaction is employed a solvent such as dimethylformamide, hexamethylphosphoric triamide, acetone, methylethylketone, tetrahydrofuran or dioxane. The reaction temperature ranges from about 50°C to about 150°C, and the reaction time is about 1 to 6 hours.

When the protective group in the protected carboxyl group in the compound obtained by the condensation of the compounds (V) and (VI) is other than the group

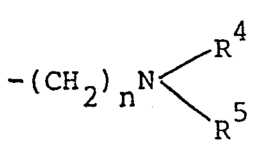 , the protective group is removed upon

necessity.

In the reaction for producing the compound (II) wherein $R^3$ is a carboxyl by removing the protective group $R^6$, reaction conditions for removing $R^6$ vary with the kinds of the protective group $R^6$. For example, when $R^6$ is tetrahydropyranyl group, the reaction is conducted in e.g. tetrahydrofuran-water or acetic acid in the presence of an acid e.g. hydrochloric acid, p-toluenesulfonic acid, etc. at about 0 to 150°C, preferably at about 0 to 50°C, more preferably around room temperature for about 30 minutes to 5 hours. When $R^6$ is benzyloxymethyl or phenacyl, the reaction can be conducted by means of catalytic reduction using e.g. palladium as the catalyst at about 0 to 150°C, preferably at about 0 to 50°C, more preferably at about room temperature for about 1 to 10 hours. When $R^6$ is t-butyl or trityl, the reaction can be conducted in the presence of an acid e.g. hydrochloric acid, trifluoroacetic acid, formic acid, p-toluenesulfonic acid,. etc. at temperatures of about 0 to 150°C, preferably ranging from those under ice-cooling (about 0 to 5°C) to about 50°C for about 1 to 5 hours.

The compound (I) is produced also by subjecting a compound of the formula:

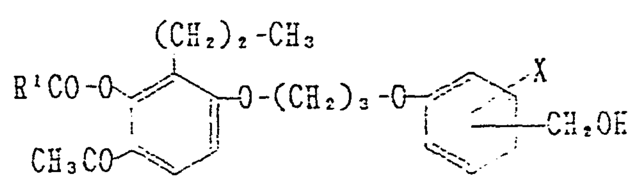

(XXIII)

wherein $R^1$ and X are of the same meanings as defined above, to oxidation.

The reagent to be employed for the reaction is exemplified by potassium permanganate, silver (I) oxide or nickel peroxide. In the reaction, about 1 to 4 equivalents of an oxidizing agent is employed relative to one equivalent of the compound (XXIII). The solvent to be employed is a conventional organic solvent, exemplified by acetic acid, acetone, benzene, acetonitrile, water or a mixture thereof. Depending on the case, the reaction can be conducted by a two-phase reaction with water. The reaction temperature is normally about 0 to 150°C and is preferably around room temperature, while it may be suitably chosen from those ranging from room temperature to the boiling point of the solvent employed. The reaction time depends on the oxidizing agent employed, and is preferably about 0.5 to 5 hours.

The compound (XXIII) is prepared by reacting the compound (V) with a compound of the formula:

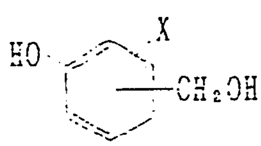

(XXIV)

wherein X is of the same meaning as defined above. The reaction conditions and reagents to be employed in this reaction are the same as those employed in the production of the compound (IV), which are mentioned hereinafter.

The compound obtained by the above method can be recovered from the reaction mixture and purified by a *per se* known process e.g. recrystallization or chromatography.

In each reaction step, the starting compound may be used in the form of a salt, and the end product may be recovered in the form of a salt. As preferable salts of the starting compounds as well as the end products in the method of this invention, pharmacologically acceptable ones are preferable, and these are exemplified by an alkali metal salt such as sodium salt or potassium salt, and an inorganic or organic acid salt such as hydrochloride, sulfate, phosphate, fumarate, maleate or oxalate.

The starting compound (IV) of the method of this invention can be prepared by allowing a starting compound (V) to be described later to react with a compound of the formula:

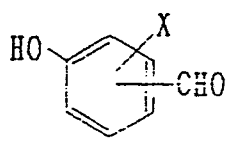

(X)

wherein X is of the same meaning as defined above under the conditions analogous to those employed in the preparation of the compounds (II) and (III).

The starting compound (V) can be prepared by allowing a known compound (IX) to react with an acylating agent of the formula:

$R^1CO-Z$ (XI) [wherein $R^1$ is of the same meaning as defined above and Z stands for a leaving group at the acylation.] The leaving group shown by the symbol Z is exemplified by an acyloxy group such as acetoxy or propionyloxy, and a halogen atom such as chlorine or bromine.

In the reaction for preparing a compound (V) by allowing a compound (IX) to react with an acylating agent (XI),

about 2 to 30 equivalents of the acylating agent (XI) is employed relative to one equivalent of the compounds (IX). The reaction is conducted preferably in the presence of a base e.g. pyridine, 4-dimethylaminopyridine and triethylamine. The solvent to be employed is exemplified by chloroform, dichloromethane, dimethylformamide, tetrahydrofuran and dioxane, and, in general, an excess volume of pyridine is employed taking the role of a solvent as well.

The starting compound (VIII) can be prepared by the following reaction steps.

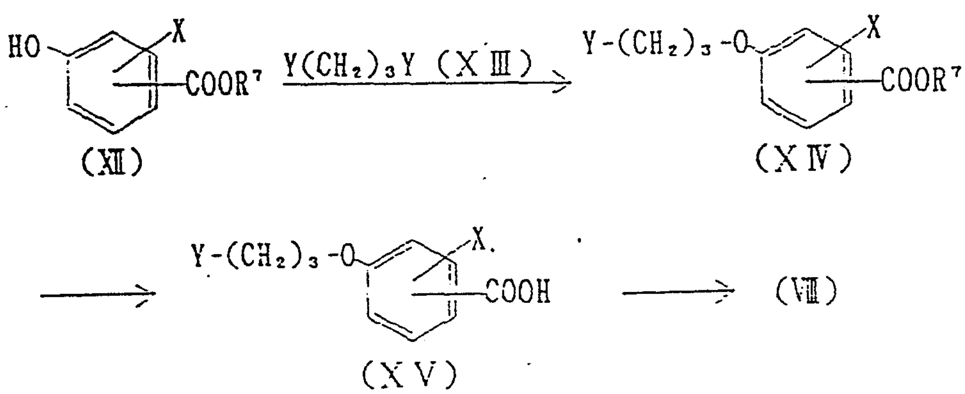


(XII) (XIV) (XV) (VIII)

A compound (XIV) can be prepared by allowing a compound of the formula (XII) [wherein X is of the same meaning as defined above and $R^7$ stands for a lower alkyl e.g. methyl and ethyl] to react with a compound of the formula $Y(CH_2)_3Y$ (XIII) [wherein Y is of the same meaning as defined above, and the two Ys may be the same or different]. As the conditions for the above reaction are employed those for preparing a compound (III) from compounds (VII) and (VIII) or from compounds (IX) and (VI'). A compound (XIV) can be led to a compound (XV) by hydrolysis. The

hydrolysis is conducted usually under alkaline conditions, and an excess amount of e.g. sodium hydroxide or potassium hydroxide is employed, and as a solvent, an aqueous methanol or an aqueous ethanol is employed. Usually, the reaction temperature ranges from room temperature to the boiling point of the solvent and the reaction time ranges from 0.5 to 5 hours. Then, the compound (XV) is led to a reactive derivative, which is allowed to react with a compound of the formula:

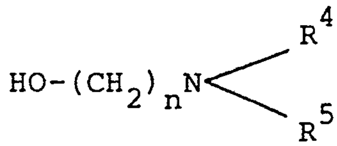

(XVI)

wherein $R^4$, $R^5$ and n are of the same meanings as defined above, or a compound (XV) or its salt is allowed to react with a compound of the formula:

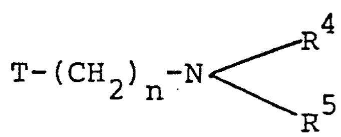

(XVII)

wherein $R^4$, $R^5$ and n are of the same meanings as defined above, and T stands for a leaving group at the esterification, to give a compound of the formula (VIII).

The reactive derivative of a compound (XV) is exemplified by an acid halide, the halogen of which is exemplified by chlorine, bromine and iodine.

The leaving group at the esterification, which is represented by the symbol T, is exemplified by halogen (e.g. chlorine, bromine and iodine), p-toluenesulfonyloxy, methanesulfonyloxy and an hydroxyl group.

When the reactive derivative of a compound (XV) is an acid halide, it can be obtained by allowing the compound (XV) or a salt thereof to react with a halogenating agent.

The halogenating agent to be employed when a compound (XV) or a salt thereof is subjected to halogenation is exemplified by thionyl chloride, phophoryl chloride, thionyl bromide, phosphorus pentachloride, phosphorus trichloride,

phosphorus oxychloride and phosphorus tribromide. The compound (XV) or a salt thereof is allowed to react with the halogenating agent in a solvent e.g. chloroform, dichloromethane, tetrachloroethane, tetrahydrofuran or dioxane at about 80 to 120°C for about 0.5 to 12 hours.

In the reaction of an acid halide of the compound (XV) with a compound (XVI), about 1 to 3 equivalents of the latter is employed relative to one mole of the acid halide. The solvent to be employed is exemplified by acetone, chloroform, dichloromethane, dimethylformamide, tetrahydrofuran and dioxane. The reaction is preferably conducted in the presence of a base e.g. triethylamine, pyridine, 4-dimethylaminopyridine and dimethylaniline. The reaction temperature is in the range of from about 15°C to 80°C, and the reaction time is about 1 to 10 hours.

In the process where the compound (XV) or its salt is allowed to react with the compound (XVII), when the compound (XVII) wherein the symbol T stands for halogen or p-toluenesulfonyloxy or methanesulfonyloxy is employed for the esterification, salts of the compound (XV) are exemplified by those of sodium, potassium, silver and tributylammonium, and about 1 to 10 equivalents of the compound (XVII) is employed relative to one equivalent of the compound (XV). The reaction is conducted preferably in the presence of a base e.g. triethylamine, pyridine, dimethylaniline and 4-dimethylaminopyridine. The solvent for this reaction is exemplified by dimethylformamide, hexamethylphosphoric triamide, acetone, methylethylketone, tetrahydrofuran and dioxane. The reaction temperature is in the range of from about 100°C to 150°C, and the reaction time is about 1 to 6 hours. The starting compound (VI) [which includes compound (VI')] can be prepared by the following reaction steps.

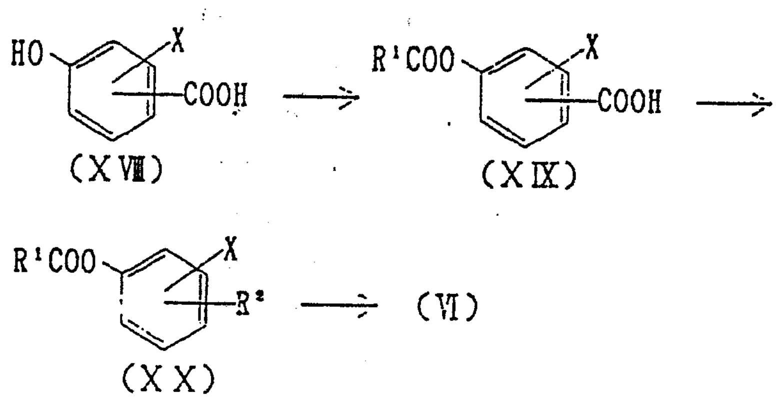

(XVIII) (XIX) (XX) (VI)

A compound of the formula (XVIII) [wherein X is of the same meaning as defined above] is subjected to acylation to lead to a compound representable by the formula (XIX) [wherein X and $R^1$ are of the same meanings as defined above]. The acylation is conducted by employing an acid anhydride under reaction conditions including the reaction time analogous to those for preparing the compound (V).

When the protective group $R^6$ is a group of the formula

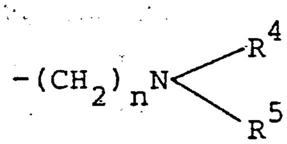

, the compound (XIX) is led to an acid halide, and the acid halide is reacted with an alcohol derivative of the protective group to give a compound of the formula (XX). The reaction conditions for leading a compound (XIX) to an acid halide are exemplified by those for preparing an acid halide from the compound (XV).

As the alcohol derivative of the protective group employed in the above-mentioned reaction is mentioned a compound (XXI) of the formula:

$$HO-R^6 \qquad (XXI)$$

wherein $R^6$ is of the same meaning as defined above. For the reaction of the above-mentioned acid halide with the alcohol derivative (XXI), about 1 to 4 equivalents of the latter is employed relative to one equivalent of the former.

The solvent to be employed is exemplified by acetone, chloroform, dichloromethane, tetrahydrofuran, dioxane, acetonitrile, etc. The reaction is conducted preferably in the presence of a base. The base is exemplified by triethylamine, pyridine, 4-dimethylaminopyridine, dimethylaniline, etc. The reaction temperature is in the range of about 15 to 80°C, and the reaction time is in the range of about 1 to 10 hours. Furthermore, the compound (XX) is produced by reacting the compound (XIX) with a halide of the protecting group.

As a preferable example of the halide of the protecting group may be mentioned a compound of the formula:

$$Z\text{-}R^6 \qquad \text{(XXII)}$$

wherein Z is a halogen atom and $R^6$ is of the same meaning as defined above. The halogen atom shown by Z is exemplified by chlorine, bromine, iodine, etc.

The amount of the compound (XXII) in the above reaction is about 1 to 4 equivalents relative to one equivalent of the compound (XIX). The reaction is conducted preferably in the presence of a base such as triethylamine, pyridine, dimethylaniline, etc. For the reaction is employed a solvent such as dimethylformamide, hexamethylphosphoric triamide, tetrahydrofuran, dioxane, acetonitrile, etc. The reaction temperature is in the range of about 70 to 150°C, and the reaction time is about 1 to 6 hours.

In the reaction for preparing the compound (XX) wherein the protective group $R^6$ in the protected carboxyl group $R^2$ is tetrahydropyranyl, t-butyryl, trityl, benzyl, benzyloxymethyl or phenacyl, reaction conditions for preparing the compound (XX) vary with the kinds of the protective group.

In said reaction, when $R^6$ is for example a tetrahydropyranyl group, dihydropyran of about 1 to 5 equivalents

relative to one equivalent of the compound (XIX) is employed, and the reaction is allowed to proceed at temperatures within the range of from those under ice-cooling to room temperature in the presence of a catalytic amount of an acid for about 10 minutes to 5 hours to give the compound (XX). The solvent to be employed is exemplified by dichloromethane, chloroform, acetonitrile, tetrahydrofuran, etc., and the acid is exemplified by p-toluenesulfonic acid, sulfuric acid, etc.

When $R^6$ is a benzyl group or t-butyl group, a compound (II') is made into an acid halide by a reaction analogous to the above mentioned process for preparing an acid halide, then the acid halide is allowed to react with benzyl alcohol or t-butyl alcohol to give the compound (XX).

When $R^6$ is a trityl group or benzyloxy methyl group, a compound (XIX) is first made into a salt of sodium, potassium or silver, etc., and the salt is allowed to react with e.g. trityl bromide or benzyloxymethyl chloride to give the compound (XX). When $R^6$ is a phenacyl group, e.g. phenacyl bromide is allowed to react with a compound (XIX) in the presence of e.g. triethylamine to give the compound (XX). The solvent to be employed for the reaction is exemplified by benzene, hexane, chloroform, dichloromethane, ethyl acetate, tetrahydrofuran, acetonitrile, etc.

For any of the above-mentioned reactions, the reaction temperature may be adequately chosen from those under ice-cooling to those about the boiling point of the solvent then employed, and the reaction time ranges from about 1 to about 5 hours.

The compound (XX) is led to a compound (VI) by subjecting it to a reaction in aqueous ammonia, a solution of potassium carbonate or sodium carbonate in water, methanol or ethanol or a mixture of any of these solutions at a temperature of under ice-cooling to about 50°C for

about 0.1 to 5 hours.

The end products (I), (II) and (III) [hereinafter, it is abbreviated as "compound (I) - (III)"] of the method of this invention have a remarkable antagonistic action on the slow reacting substance of anaphylaxis (SRS-A) which is a chemical mediator known to induce a contraction of bronchial smooth muscle.

SRS-A is produced by various stimuli such as immune reactions and has been considered to be a potent mediator of bronchospasm in immediate allergy such as allergic asthma. SRS-A consists of leukotriene C(LTC), leukotriene D(LTD), leukotriene E(LTE), and it is known that LTD and LTC are substantially equivalent in activity on the human bronchial muscle

and that LTD is superior to LTC in constrictive effect on the guinea pig ileum [S.E. Dahlen et al., Nature 288, 484 (1980); R.A. Lewis et al., Biochemical and Biophysical Research Communication 96, 271 (1980)]. The antagonistic effect of drugs against SRS-A can be investigated using the guinea pig ileum [R.A. Appleton et al., Journal of Medicinal Chemistry 20, 371 (1977)] and since SRS-A is a mixture of LTC, LTD, etc. and the ratio thereof is indefinite, it is desirable to use a synthetic SRS-A in the investigation of antagonistic activity.

The present inventors studied the antagonistic action of compound (I) against SRS-A using a synthetic $LTD_4$ in the following manner, and found that, against the bronchoconstriction in guinea pigs due to an intravenous administration of synthetic leukotriene $D_4$ ($LTD_4$), some of the object compounds of the method of the present invention when administered orally one hour before $LTD_4$ dosing displayed a remarkable inhibitory effect superior to the control compound.

(1) Test method

Guinea pigs of the Hartley strain, both male and female, with body weights of about 400 g were assigned to groups of 6-10 individuals, and the bronchoconstriction due to $LTD_4$ was measured according to the method of

Konzett-Rössler, [Konzett, H. and Rössler, R.: Naunyn-Schmidebergs Archiv für Experimentelle Pathologie und Pharmakologie, 195, 71-74 (1940).] Each guinea pig was fixed in a supine position under urethane anesthesis (1.5 g/kg, intraperitoneal) and the trachea was incised and connected to an artificial respiration apparatus, Rodent Respirator Model 680 [Harvard Apparatus Company, U.S.A.] via a cannula. The branch tube of this tracheal cannula was connected to a Bronchospasm Transducer Model 7020 [Ugobasil Biological Research Apparatus, Italy]. Under the conditions of 4 to 7 ml of air per stroke, 70 strokes per minute and a lung loading pressure of 10 $cmH_2O$, the volume of overflowing air was recorded on Rectigraph-8S (San-ei Sokki Ltd., Japan) via a transducer. After administration of gallamine triethiodide (1 mg/kg, i.v.), a solution of $LTD_4$ in physiological saline (10 μg/kg) was intravenously administered and the bronchoconstriction elicited thereby was recorded for 15 minutes. The compound was used as suspended in a 5% solution of gum arabic or dissolved in water, and administered orally in a volume of 0.2 ml per 100 g body weight one hour before $LTD_4$ loading. $LTD_4$ was administered through a cannula inserted into the jugular vein. $LTD_4$ was used as dissolved in physiological saline, which was taken from a stock stored in methanol (1 mg/1 mℓ methanol) at -70°C.

(2) Result

| Compound *1 | *2 $ID_{50}$(p.o.) (μmol/Kg) |
|---|---|
| A | 47 |
| B | 49 |
| C | 41 |
| D | 49 |
| E | 52 |
| Control *3 | 146 |

*1 A: Compound of Example 1
B: Compound of Example 6(3)
C: Compound of Example 4
D: Compound of Example 6(4)
E: Compounds of Examples 5 and 7

*2 $ID_{50}$ (50% inhibitory dose): Each value was calculated from the relation between dosage and the inhibition rate of overflowing volume (in percentage) from the respiratory tract at the time when the response was maximal, i.e. 30 seconds after the administration of $LTD_4$.

*3 Control compound: The compound disclosed in the specification of European Patent Application Publication No. 80,371, shown by the formula:

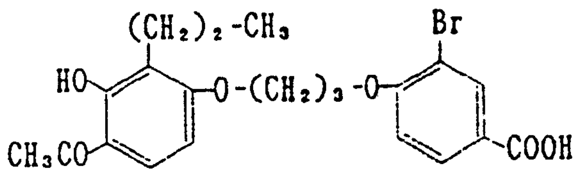

The acute toxicity of compound A, compound C or compound E in mice was found to be as follows.

(1) Method

Five 5-week-old male mice of Jcl:ICR strain, weighing about 30 g each, were used. Compound A, C or E was suspended in a 5% solution of gum arabic and administered orally at the level of 0.2 ml per 10 grams body weight.

(2) Results

The oral administration of compound C or E in a dose of 2000 mg/kg and of compound A in a dose of 500 mg/kg caused no symptoms that could be attributable to these compounds. Autopsy after 7 days did not reveal any abnormalities.

From the foregoing, the toxicity of the object compounds(I)-(III) of the method of this invention is considered to be low.

It will thus be apparent that the compounds (I)-(III) according to this invention are useful in the treatment of diseases due to SRS-A, such as asthma, hay fever, chronic bronchitis, allergic diseases of the eye,

allergic diseases of the stomach and intestines, cardiovascular disturbances, allergic dermatitis and other inflammatory diseases. For example, as an antiasthmatic or antiinflammatory drug, the compounds (I)-(III) or salts thereof can be administered orally or parenterally to mammalian animals (e.g. mouse, rat, guinea pig, man) in a daily dose of about 1 to 20 mg/kg.

For oral administration, the compounds (I)-(III) or salts thereof can be formulated with a pharmaceutically acceptable carrier, excipient or diluent (e.g. lactose, starch, cellulose derivatives, stearic acid, magnesium stearate, sucrose, gelatin, gum arabic) and processed into such dosage forms as tablets, capsules, granules, troches, liquid, syrup, etc. For parenteral administration, the compounds (I)-(III) or salts thereof can be formulated with pharmacologically acceptable vehicles, excipients or diluents (e.g. white petrolatum, hydrophilic ointment bases, oleaginous bases, glyceride, polyethlene glycol, etc.) and processed into ointments, suppositories, aerosols, inhalants, injections, etc. These dosage forms may be produced by the established pharmaceutical procedures.

According to the method of the present invention, the compounds (I)-(III) displaying remarkably excellent pharmaceutical effects, can be produced advantageously on an industrial scale.

The following Reference Examples and Examples illustrate the present invention in more detail.

Reference Example 1

A mixture of 2,4-dihydroxy-3-propylacetophenone (2.7 g), 1,3-dibromopropane (2.8 ml) and potassium carbonate (1.9 g) in acetone (50 ml) was refluxed for 3 hours. Insolubles were filtered off, and the filtrate was concentrated. The resultant syrup was purified by means of a silica gel flash chromatography (chloroform-hexane = 1:1) to give 3-(4-acetyl-3-hydroxy-2-propylphenoxy)propylbromide (2.9 g) as a colorless and transparent syrup.

NMR($CDCl_3$)δ: 0.92(3H,t,J=7Hz), 1.30-1.70(2H,m), 2.33 (2H,m), 2.54(3H,s), 2.63(2H,t,J=6Hz), 3.60(2H,t,6Hz), 4.18 (2H,t,J=6Hz), 6.43(1H,d,J=9Hz), 7.55(1H,d,9Hz)

Reference Example 2

In methylene chloride (30 ml) were dissolved 3-(4-acetyl-3-hydroxy-2-propylphenoxy)propylbromide (2.8 g), triethylamine (6.2 ml) and dimethylaminopyridine (10 mg). To the solution was added dropwise acetic anhydride (4.2 ml) under ice-cooing. The reaction was allowed to proceed for 3 hours, to which was added methanol (5 ml). The mixture was left standing for 10 minutes. The solvent was evaporated off to leave a syrup, which was

dissolved in chloroform. The solution was washed with water, dried and concentrated. The resultant syrup was purified by means of a silica gel flash chromatography (chloroform) to give 3-(3-acetoxy-4-acetyl-2-propylphenoxy)propylbromide (2.7 g) as a colorless and transpararent syrup.

NMR($CDCl_3$)$\delta$: 0.93(3H,t,J=7Hz), 1.30−1.70(2H,m), 2.36 (3H,s), 2.50(3H,s), 2.20~2.70(4H,m), 3.59(2H,t,J=6Hz), 4.29(2H,t,J=6Hz), 6.79(1H,d,J=9Hz), 7.71(1H,d,J=9Hz)

Reference Example 3

A mixture of 3-(3-acetoxy-4-acetyl-2-propylphenoxy)-propylbromide (2.3 g), 3-bromo-4-hydroxybenzaldehyde (1.29 g), potassium iodide (1.07 g) and potassium carbonate (0.89 g) was heated in dimethylformamide (30 ml) at 70-80°C for 1.5 hours under stirring. To the reaction mixture was added chloroform (100 ml), and insolubles were filtered off. The filtrate was washed with water, dried and concentrated to give a brownish syrup, which was purified by means of a silica gel flash chromatography. The thus-purified syrup was triturated with isopropyl ether to cause crystallization. The crystals were recrystallized from aqueous ethanol to give 4-[3-(3-acetoxy-4-acetyl-2-propylphenoxy)propoxy]-3-bromobenzaldehyde (2.28 g) as pale yellow crystals, m.p. 120-121°C.

Reference Example 4

A suspension of 4-acetoxy-3-bromobenzoic acid (1.5 g) in thionyl chloride (6 ml) was refluxed for one hour, followed by concentration to dryness. The residue was dissolved in toluene and the toluene was evaporated off. This procedure was carried out twice. The resultant oily substance was dissolved in acetone (10 ml), to which was added dropwise under ice-cooling 3-dimethylaminopropanol (0.8 ml). The mixture was stirred at room temperature for 30 minutes. The resultant crystals were collected by filtration, and were recrystallized from ethanol-ether to give 3-dimethylaminopropyl 4-acetoxy-3-bromobenzoate·hydrochloride (1.8 g) as colorless crystals, m.p. 163-167°C.

Reference Example 5

To a solution of 3-dimethylaminopropyl 4-acetoxy-3-bromobenzoate (380 mg) in methanol (4 ml) was added 2 N aqueous ammonia (0.75 ml). The reaction was allowed to proceed at room temperature for 15 minutes, then the solvent was evaporated off. The residue was crystallized from acetone and recrystallized from methanol-ether to give 3-dimethylaminopropyl 3-bromo-4-hydroxybenzoate·hydrochloride (200 mg) as colorless crystals, m.p. 186-190°C.

Reference Example 6

(1) In 60 ml of dichloromethane was dissolved 2.591 g of 4-acetoxy-3-bromobenzoic acid. After the addition of two drops of conc. sulfuric acid to the solution, isobutene was introduced into the mixture under ice-cooling for 30 minutes. The flask was stoppered tightly and kept standing for 2 days at room temperature. Nitrogen gas was introduced into the mixture so as to remove isobutene, and the resultant was washed with water, a saturated aqueous solution of sodium hydrogen carbonate, 1N hydrochloric acid and then water in the order mentioned. The resultant was dried with magnesium sulfate, and the solvent was removed by evaporation to give 2.55 g of a brown oily product.

The oily product (1.9 g) was dissolved in 24 ml of ethanol, and 9 ml of 1N sodium hydroxide was added to the solution under stirring at room temperature. After further stirring for one hour, the pH of the mixture was adjusted to 7 with acetic acid. Ethanol was removed by evaporation under reduced pressure and to the resultant was added water. The pH of the mixture was adjusted to 5 with acetic acid. The mixture was subjected to extraction with chloroform. The extract was dried with magnesium sulfate, and chloroform was removed by evaporation under reduced pressure. The resultant was subjected to purification with flash chromatography using silica gel, and subjected to

recrystallization from hexane to give 0.76 g of tert-butyl 3-bromo-4-hydroxybenzoate as colorless needles. m.p. 106-108°C.

(b) A mixture of 2,4-dihydroxy-3-propylacetophenone (85.5 g), anhydrous potassium carbonate (122 g), acetone (700 ml), and 1-bromo-3-chloropropane (87 ml) was refluxed for 8 hours under stirring, and cooled in an ice bath. An insoluble material was removed by filtration and washed with ethyl acetate (150 ml). The filtrate was evaporated to dryness and the residue was dissolved in dichloromethane (500 ml). N,N-Dimethylaminopyridine (0.5 g) and triethylamine (185 ml) were added to the ice-cooled solution. Then, to the solution was added dropwise acetic anhydride (125 ml) at 5-10°C and stirring was continued for 30 minutes. The icebath was removed and the solution was stirred at room temperature for 3 hours. Water (200 ml) was added to the solution keeping the temperature below 20°C, and the mixture was stirred at about 15°C for 15 minutes. To the solution was added dichloromethane (300 ml) and water (300 ml).

The separated dichloromethane layer was washed with water (500 ml), dilute hydrochloric acid (500 ml), a saturated solution of sodium chloride in water (100 ml), in this order, and then dried (sodium sulfate). The solution was evaporated to dryness and the residue was distilled by the use of Kugelrohr (Aldrich, U.S.A.) to give 3-(3-acetoxy-4-acetyl-2-propylphenoxy)propyl chloride as a yellow oil (132.5 g). b.p. 160-220°C/1.2-1.5 mmHg.

NMR ($CDCl_3$) δ: 0.92(3H,t,J=7Hz), ca. 1.50(2H,m), 2.10-2.65(4H,m), 2.39(3H,s), 2.56(3H,s), 3.75(2H,t,J=6Hz), 4.20(2H,t,J=6Hz), 6.79(1H,d,J=9Hz), 7.71(1H,d,J=9Hz)

## Reference Example 7

A mixture of methyl 3-bromo-4-hydroxybenzoate (44 g), acetone (300 ml), potassium carbonate (25 g) and 3-bromo-1-chloropropane (31 g) was refluxed for 16 hours. Then, inorganic salts were filtered off, and the filtrate was concentrated. The concentrate was purified by means of a silica gel column chromatography (hexane-ethyl acetate ester=4:1) to give methyl 3-bromo-4-(3-chloropropoxy)benzoate as crystals (50 g), m.p. 67-68°C.

## Reference Example 8

A mixture of methyl 3-bromo-4-(3-chloropropoxy)-benzoate (2.8 g), methanol (10 ml) and 2 N sodium hydroxide (5 ml) was stirred at 50-60°C for one hour. Then, the methanol was evaporated off, and the residue was made acidic with dilute hydrochloric acid. Precipitating crystals were collected by filtration, washed with water, then dissolved in chloroform. The solution was subjected to a silica gel column chromatography. Elution was conducted with chloroform. The eluate was concentrated. To the concentrate was added hexane. Precipitating crystals were collected by filtration to give 3-bromo-4-(3-chloropropoxy)benzoic acid (2.5 g). m.p. 149-150°C.

## Reference Example 9

A solution of 3-bromo-4-(3-chloropropoxy)benzoic acid (1.2 g), chloroform (5 ml) and

thionyl chloride (1 ml) was refluxed for one hour. The solvent was evaporated off from the resultant, and the residue was dried on sodium hydroxide and then dissolved in acetone (20 ml). To the solution was added 1-(2-hydroxyethyl)pyrrolidine (500 mg), to which was added triethylamine (2 ml). The mixture was stirred at room temperature for one hour. Resultant precipitates were filtered off and the solvent was evaporated off. The residue was purified by means of a silica gel column chromatography (eluent: ethyl acetate) to give 2-pyrrolidinoethyl 3-bromo-4-(3-chloropropoxy)benzoate (1.1 g) as an oily substance.

NMR($CDCl_3$)$\delta$: 1.40-1.90(4H,m), 2.10-2.90(8H,m), 3.77 (2H,t,J=6Hz), 3.90-4.50(4H,m), 6.83(1H,d,J=8Hz), 7.85(1H, dd,J=2 and 8Hz), 8.10(1H,d,J=2Hz)

Reference Example 10

By the procedure of Reference Example 9, the following compounds were prepared.

(1) 2-morpholinoethyl 3-bromo-4-(3-chloropropoxy)-benzoate (oily product)

NMR($CDCl_3$)$\delta$: 2.10-2.90(8H,m), 3.55-4.00(6H,m), 4.10-4.55(4H,m), 6.87(1H,d,J=8Hz), 7.90(1H,dd,J=2 and 8Hz), 8.15(1H,d,J=2Hz)

(2) 3-dimethylaminopropyl 3-bromo-4-(3-chloro-

propoxy) benzoate (oily product)

NMR ($CDCl_3$) $\delta$: 1.60-2.60(6H,m), 2.23(6H,s), 3.81(2H,t, J=6Hz), 4.29(4H,q,J=6Hz), 6.86(1H,d,J=8Hz), 7.90(1H,dd,J=2 and 8Hz), 8.15(1H,d,J=2Hz)

(3) 4-(N,N-dimethylamino)butyl 3-bromo-4-(3-chloropropoxy)benzoate (oily product)

(4) dimethylaminoethyl 3-bromo-4-(3-chloropropoxy)-benzoate (oily product)

(5) 2-(4-methyl-1-piperazinyl)ethyl 3-bromo-4-(3-chloropropoxy)benzoate (oily product).

## Example 1

To a solution of 4-[3-(3-acetoxy-4-acetyl-2-propylphenoxy)propoxy]-3-bromobenzaldehyde (2.39 g) in acetone (50 ml) was added acetic acid (5 ml). To the mixture, while sufficiently stirring at room temperature, was added pulverized potassium permanganate (1.58 g) by portions. The reaction was allowed to proceed while stirring for one hour. The solvent was evaporated off under reduced pressure, and the concentrate was subjected to extraction with chloroform. Insolubles were filtered off, and the filtrate was washed with a dilute aqueous solution of hydrogen peroxide then with water. The resultant was dried and concentrated to give a colorless and transparent

syrup. Crystallization from aqueous alcohol gave 4-[3-(3-acetoxy-4-acetyl-2-propylphenoxy)propoxy]-3-bromobenzoic acid (2.0 g), m.p. 155-156°C.

Example 2

In dimethylformamide (30 ml), 3-(3-acetoxy-4-acetyl-2-propylphenoxy)propylbromide (1.6 g) was allowed to react with 3-dimethylaminopropyl 3-bromo-4-hydroxybenzoate·hydrochloride (1.7 g), potassium iodide (0.83 g) and potassium carbonate (0.7 g) at 60-70°C for 6 hours while stirring. Insolubles were filtered off, and the filtrate was concentrated. The resulting syrup was purified by means of a flash chromatography using silica gel to give 3-dimethylaminopropyl 4-[3-(3-acetoxy-4-acetyl-2-propylphenoxy)propoxy]-3-bromobenzoate (1.8 g). The product was dissolved in ether, to which was added oxalic acid (250 mg) to give a crystalline oxalate (1.8 g) of said compound. m.p. 141-142°C.

Example 3

(1) To a mixture of 1.565 g of 3-(3-acetoxy-4-acetyl-2-propylphenoxy)propyl chloride, 375 mg of sodium iodide, 503 mg of sodium carbonate and 1.229 g of tert-butyl 3-bromo-4-hydroxybenzoate was added 10 ml of N,N-dimethylformamide, and the mixture was vigorously stirred at 80 to 85°C for 6 hours. After cooling, to the reaction mixture was added 25 ml of ethyl acetate and 25 ml of water, and the aqueous layer and organic layer were separated from each other.

The aqueous layer was subjected to extraction with 10 ml of ethyl acetate. The ethyl acetate layers were combined and dried with 25 ml of 2% hydrochloric acid and then with 25 ml of a saturated aqueous solution of NaCl. The solution was dried with sodium sulfate, and ethyl acetate was removed by evaporation under reduced pressure to give 3 g of tert-butyl 4-[3-(3-acetoxy-4-acetyl-2-propylphenoxy)propoxy]-3-bromobenzoate as a pale brown oily product.

NMR ($CDCl_3$) δ: 0.88(3H,t,J=7Hz), 1.32-1.70(2H,m), 1.57(9H,s), 2.20-2.65(4H,m), 2.33(3H,s), 2.45(3H,s), 4.13-4.40(4H,m), 6.78(1H,d,J=9Hz), 6.86(1H,d,J=8Hz), 7.67(1H,d,J=9Hz), 7.88(1H,dd,J=2 and 8Hz), 8.13(1H,d,J=2Hz)

(2) In 10 ml of dioxane was dissolved 1.5 g of tert-butyl 4-[3-(3-acetoxy-4-acetyl-2-propylphenoxy)propoxy]-3-bromobenzoate. To the solution was added under stirring at 20°C, 10 ml of a

dioxane solution (2N) of hydrogen chloride which was previously ice-cooled. The mixture was further stirred for 30 minutes at room temperature, and then to the mixture was added 10 ml of the dioxane solution of hydrogen chloride. The mixture was stirred for 1.5 hours, and concentrated under reduced pressure. The residue was dissolved in 30 ml of chloroform, and the solution was washed with 30 ml of water and then dried with magnesium sulfate. Chloroform was removed by evaporation under reduced pressure, and the residue was dissolved in isopropyl ether. The solution was treated with activated charcoal, and to the solution was added hexane for crystallization. The crystals were collected by filtration, subjected to purification by silica gel column chromatography, and then recrystallized from aqueous ethanol to give 0.35 g of 4-[3-(3-acetoxy-4-acetyl-2-propylphenoxy)propoxy]-3-bromobenzoic acid as colorless crystals. m.p. 154-156°C

Example 4

A mixture of 2,4-dihydroxy-3-propylacetophenone (1.0 g), 2-morpholinoethyl 3-bromo-4-(3-chloropropoxy) benzoate (1.35 g), dimethylformamide (10 ml), potassium carbonate (1.0 g) and potassium iodide (about 10 mg) was stirred at 90-100°C for about 21 hours, and insolubles were filtered off, followed by concentration of the filtrate. The concentrate was purified by means

of a silica gel column chromatography (hexane-ethyl acetate = 1:1), and the eluate was crystallized from hexane. Recrystallization from ethanol-water gave crystals (600 mg) of 2-morpholinoethyl 4-[3-(4-acetyl-3-hydroxy-2-propylphenoxy)propoxy]-3-bromobenzoate, m.p. 98-99°C.

Example 5

A mixture of 2,4-dihydroxy-3-propylacetophenone (216 mg), 3-dimethylaminopropyl 3-bromo-4-(3-chloropropoxy)benzoate (210 mg), potassium iodide (100 mg), potassium carbonate (228 mg) and dimethylformamide (5 ml) was stirred at 60-70°C for 14 hours, from which was evaporated off dimethylformamide. To the residue was added ethyl acetate, and the mixture was subjected to a silica gel (30 g) column chromatography. Elution was conducted with chloroform-methanol (10:1). The eluate was concentrated, and was crystallized from hexane to give crystals (134 mg) of 3-dimethylaminopropyl 4-[3-(4-acetyl-3-hydroxy-2-propylphenoxy)propoxy]-3-bromobenzoate, m.p. 71-72°C.

Example 6

By the procedure of Example 5, the following compounds were produced.

(1) 2-pyrrolidinoethyl 4-[3-(4-acetyl-3-hydroxy-2-propylphenoxy)propoxy]-3-bromobenzoate, m.p. 79-80°C

(2) 4-(N,N-dimethylamino)butyl 4-[3-(4-acetyl-3-hydroxy-2-propylphenoxy)propoxy]-3-bromobenzoate, m.p. 69-70°C

(3) 2-dimethylaminoethyl 4-[3-(4-acetyl-3-hydroxy-2-propylphenoxy)propoxy]-3-bromobenzoate·hydrochloride·monohydrate, m.p. 38-40°C (hygroscopic)

(4) 2-(4-methyl-1-piperazinyl)ethyl 4-[3-(4-acetyl-3-hydroxy-2-propylphenoxy)propoxy]-3-bromobenzoate·dihydrochloride·monohydrate, m.p. 142-144°C.

Example 7

A mixture of 4-(3-chloropropoxy)-2-hydroxy-3-propylacetophenone (700 mg), dimethylformamide (10 ml), potassium iodide (450 mg), 3-dimethylaminopropyl 3-bromo-2-hydroxybenzoate·hydrochloride (900 mg) and potassium carbonate (500 mg) was stirred at 70-80°C for 5 hours. Insolubles were filtered off, and the filtrate was concentrated. The concentrate was subjected to chromatography using a silica gel column. Elution was conducted with ethyl acetate. Desired fractions were collected, and were concentrated, followed by recrystallization from n-hexane to give 3-dimethylaminopropyl 4-[3-(4-acetyl-3-hydroxy-2-propylphenoxy)propoxy]-3-bromobenzoate as colorless crystals (910 mg), m.p. 71-72°C.

What we claim is:

1. A method for preparing a benzoic acid derivative of the formula:

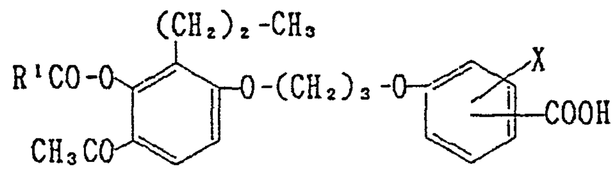

wherein $R^1$ is a lower alkyl group and X is a halogen atom, which comprises subjecting a compound of the formula:

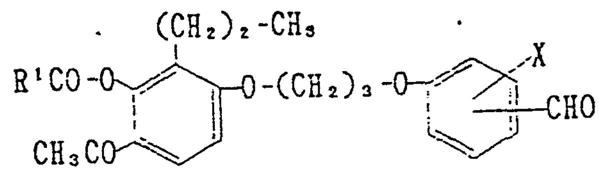

wherein $R^1$ and X are of the same meanings as defined above to oxidation.

2. A method as claimed in Claim 1, wherein $R^1$ is methyl.

3. A method as claimed in Claim 1, wherein X is bromine.

4. A method of preparing a benzoic acid derivative of the formula:

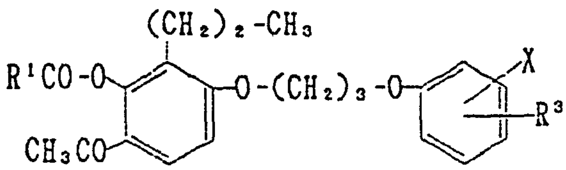

wherein $R^1$ is a lower alkyl group, $R^3$ is a carboxyl group which may be protected by a group of the formula:

$$-(CH_2)_nN\begin{matrix} R^4 \\ R^5 \end{matrix}$$ (wherein n is an integer of 2 to 4,

$R^4$ and $R^5$ independently are hydrogen or lower alkyl, and $R^4$ and $R^5$, taken together with the adjacent nitrogen atom, may form a 5- or 6-membered heterocyclic ring), and X is a halogen atom, which comprises subjecting a compound of the formula:

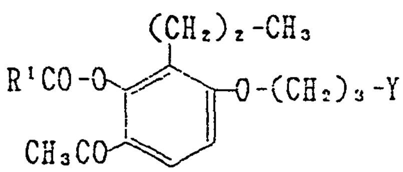

wherein $R^1$ is of the same meaning as defined above and Y is a leaving group at the condensation, with a compound of the formula:

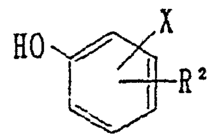

wherein $R^2$ is a protected carboxyl group and X is of the same meaning as defined above, to condensation, and then subjecting the thus obtained compound to a reaction for removing the protective group, upon necessity.

5. A method as claimed in Claim 4, wherein $R^1$ is methyl.

6. A method as claimed in Claim 4, wherein $R^3$ is carboxyl.

7. A method as claimed in Claim 4, wherein $R^3$ is a group of $-COO(CH_2)_3N\begin{matrix}CH_3\\CH_3\end{matrix}$.

8. A method as claimed in Claim 4, wherein X is bromine.

9. A method for preparing a benzoic acid derivative of the formula:

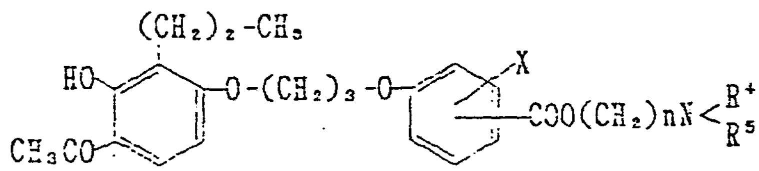

wherein n is an integer of 2 to 4, $R^4$ and $R^5$ independently are a hydrogen atom, a lower alkyl group, or, they form, together with the adjacent nitrogen atom, a 5- or 6-membered heterocyclic ring, and X is a halogen, which comprises subjecting a compound of the formula:

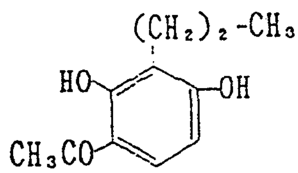

to a condensation reaction with a compound of the formula:

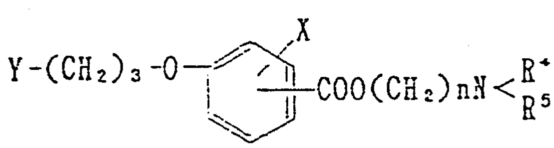

wherein n, $R^4$, $R^5$ and X are of the same meanings as defined above, and Y is a leaving group at the condensation.

10. A method for preparing a benzoic acid derivative of the formula:

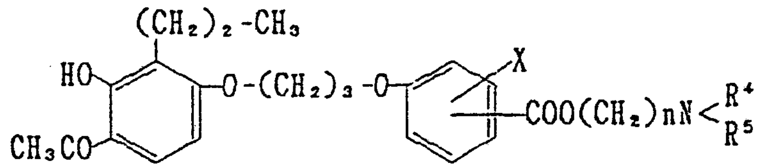

wherein n is an integer of 2 to 4, $R^4$ and $R^5$ independently are a hydrogen atom, a lower alkyl group, or, they form, together with the adjacent nitrogen atom, a 5- or 6-membered heterocyclic ring, and X is a halogen, which comprises subjecting a compound of the formula:

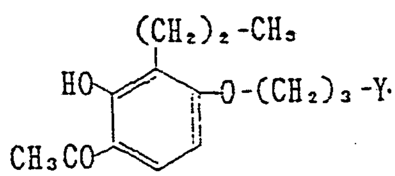

wherein Y is a leaving group at the condensation, to a condensation reaction with a compound of the formula:

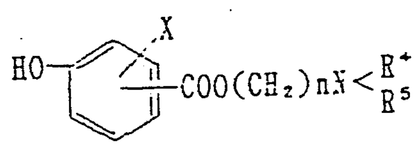

wherein n, $R^4$, $R^5$ and X are of the same meanings as defined above.